# FASCICULE DE BREVET EUROPEEN

(11) **EP 3 426 327 B1**
(45) Date de publication et mention de la délivrance du brevet: **09.07.2025**
(21) Numéro de dépôt: 17708862.2
(22) Date de dépôt: 13.02.2017
(51) Int. Cl.: A61M 5/30

(54) **DISPOSITIF D'INJECTION SANS AIGUILLE ÉQUIPÉ D'UNE BUSE D'INJECTION PERFECTIONNÉE**
NADELLOSE INJEKTIONSVORRICHTUNG MIT EINER VERBESSERTEN INJEKTIONSDÜSE
NEEDLELESS INJECTION DEVICE EQUIPPED WITH AN IMPROVED INJECTION NOZZLE

(30) Priorité: 08.03.2016 FR 1651928
(43) Date de publication de la demande: 16.01.2019
(73) Titulaire: Crossject, 21000 Dijon (FR)
(72) Inventeur: STEINBERGER, Robin, 2544 Leobersdorf (AT); VIVIEN, Gilles, 92240 Malakof (FR)
(74) Mandataire: Germain Maureau
(86) Numéro de dépôt international: PCT/FR2017/050321
(87) Numéro de publication internationale: WO 2017/153652

(56) Documents cités:
- WO-A1-2012/044259
- WO-A1-97/31665
- FR-A1- 2 853 837
- US-A- 2 667 874
- US-A- 5 061 135
- US-A- 5 875 976
- US-A1- 2002 123 717
- US-A1- 2011 176 889
- US-A1- 2015 050 102
- SASS ET AL: "DUBBEL'S TASCHENBUCH FUER DEN MASCHINENBAU, PASSAGE", DUBBELS TASCHENBUCH FUER DEN MASCHINENBAU, XX, XX, 1 January 1955 (1955-01-01), pages 602 - 605, XP002051543

## Description

L'invention concerne un dispositif d'injection sans aiguille équipé d'une buse d'injection perfectionnée.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

D'autres dispositifs d'injections sont connus des documents US5875976 et FR2853837.

Le réservoir est constitué par un tube en verre qui est inséré dans un logement tubulaire délimité par le corps du dispositif, le tube étant obturé par un piston supérieur, ou amont, et un piston inférieur, ou aval, entre lesquels est contenu le principe actif liquide.

L'extrémité libre inférieure du réservoir coopère avec une buse d'injection qui délimite au moins un canal d'injection s'étendant axialement suivant un axe d'injection.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appui la buse d'injection sur sa peau.

Le déplacement du corps dans le capot permet le déclenchement du générateur de gaz, générant un gaz sous pression qui entraîne en déplacement les pistons pour injecter le principe actif à travers la peau du patient, en passant par la buse d'injection.

On connait un dispositif d'injection qui est équipé d'une membrane déformable élastiquement de forme globalement en T**,** qui comprend un disque annulaire radial qui est interposé axialement entre l'extrémité supérieure du réservoir et un siège formé par le corps, et une partie tubulaire qui s'étend axialement dans le réservoir, depuis le disque annulaire.

La partie tubulaire de la membrane est conçue pour s'étendre axialement sous l'effet du gaz sous pression, afin d'entraîner les pistons en déplacement.

La buse d'injection est délimitée axialement par une face supérieure en appui axial sur le réservoir, et une face inférieure d'injection adaptée pour coopérer avec un opercule de fermeture.

Aussi, la buse d'injection présente un filetage externe adapté pour être vissé sur un taraudage complémentaire formé par le corps.

Ainsi, le vissage de la buse sur le corps permet de pincer axialement le disque annulaire radial de la membrane entre l'extrémité supérieure du réservoir et le siège formé par le corps, pour assurer l'étanchéité entre la membrane, le réservoir et le corps lors de l'injection.

Sous l'effet de la pression générée par le générateur de gaz, on constate parfois un glissement entre le corps et l'ensemble formé par la membrane et le réservoir.

Ce glissement désolidarise partiellement la liaison entre la membrane et le corps jusqu'à créer des fuites plus ou moins importantes perturbant aléatoirement les caractéristiques d'injection du dispositif d'injection, ces fuites génèrant des dispersions sur les performances d'injection.

La présente invention est définie par la revendication 1 et vise notamment à résoudre cet inconvénient et se rapporte pour ce faire à un dispositif d'injection sans aiguille comportant :
- un corps formant un logement qui s'étend axialement depuis un siège radial supérieur, suivant un axe d'injection,
- un générateur de gaz,
- un réservoir tubulaire qui contient un principe actif et qui s'étend axialement dans ledit logement depuis une extrémité supérieure, jusqu'à une extrémité inférieure, et
- une buse d'injection du principe actif qui est agencée à l'extrémité inférieure du réservoir, et qui présente un filetage externe adapté pour être vissé sur un taraudage complémentaire formé par le corps, le dispositif d'injection étant caractérisé en ce que le filetage de la buse présente une section globalement triangulaire, ledit filetage étant délimité par une face supérieure qui présente un premier angle par rapport à un axe radial perpendiculaire à l'axe d'injection, et une face inférieure qui présente un second angle par rapport audit axe radial, et en ce que le premier angle est supérieur au second angle.

La conception de la buse selon l'invention permet de limiter le second angle formé par la face inférieure du filetage de la buse, ce qui permet un couple de serrage plus important de la buse avant rupture de la buse ou du taraudage complémentaire formé par le corps.

Ainsi, le risque de fuite entre la membrane et le réservoir est écarté.

Selon l'invention, le filetage est du type en dent de scie, c'est-à-dire que le second angle formé par la face inférieure de la buse est non nul de sorte que ladite face inférieure forme une face de dépouille.

Une telle face de dépouille favorise le démoulage de la buse au cours de sa fabrication.

De préférence, le premier angle est d'environ 35 degrés.

Aussi, le second angle est d'environ 10 degrés. Un angle de 10 degrés est compromis intéressant qui favorise simultanément le démoulage de la buse et qui permet un couple de serrage important de la buse.

Selon une autre caractéristique, la face inférieure du filetage de la buse est en appui sur une face supérieure complémentaire du taraudage associé formé par le corps, pour reprendre les efforts axiaux de poussée transmis par la buse au cours de l'injection.

Aussi, la portion courbe qui relie deux triangles voisins formés par le filetage 72 présente un rayon de courbure d'environ 0,11 millimètre.

De plus, le pas du filetage est d'environ un millimètre.

De préférence, la buse d'injection est réalisée en moulage par injection.

De même, le corps est réalisé en moulage par injection.

Selon une autre caractéristique, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue éclatée en perspective qui illustre le dispositif d'injection selon l'invention ;
- la figure 2 est une vue en section axiale qui illustre le dispositif de la figure 1 dans une position de repos ;
- la figure 3 est une vue de détail en section axiale qui illustre l'accouplement de la buse d'injection sur le corps du dispositif d'injection de la figure 1 ;
- la figue 4 est une vue de détail en section axiale d'un arrachement qui illustre le filetage de la buse de la figure 3.

Dans la description et les revendications, pour clarifier la description et les revendications, on adoptera à titre non limitatif la terminologie longitudinal, vertical et transversal en référence au trièdre L**,** V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe B d'injection du dispositif d'injection.

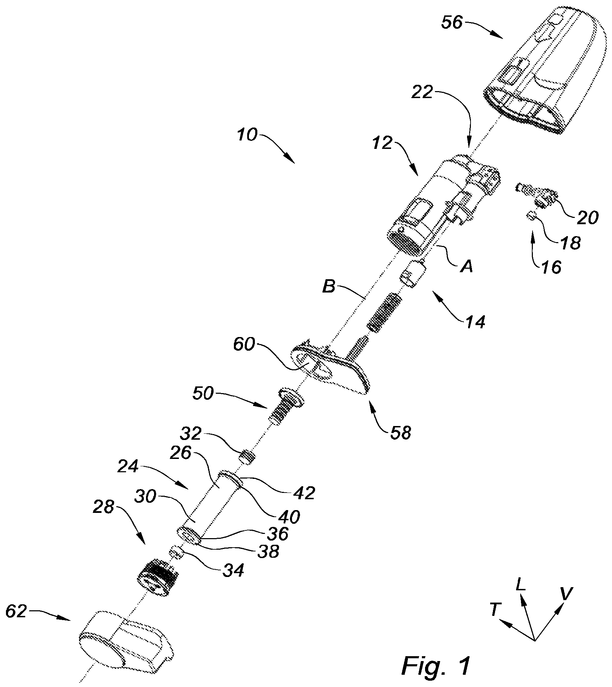

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston supérieur 32 et un piston inférieur 34 entre lesquels est contenu le principe actif 26 liquide, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

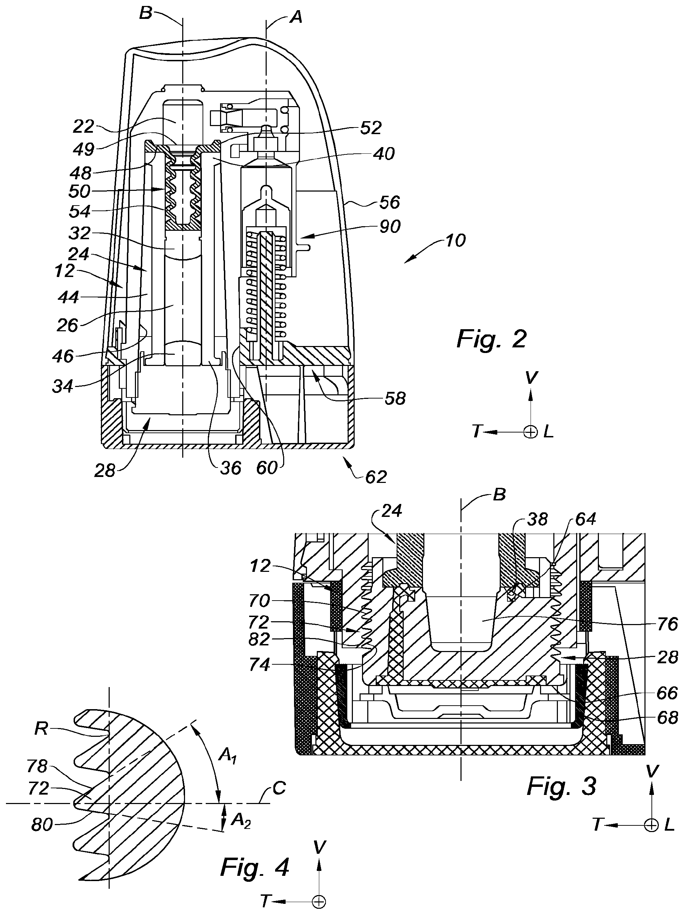

Comme on peut le voir à la figure 2, le réservoir 24 est agencé dans un logement 44 formé par le corps 12, logement 44 qui est délimité radialement par une paroi 46 tubulaire qui s'étend autour de l'axe B d'injection.

Le logement 44 s'étend axialement depuis un siège 48 radial supérieur qui est formé par le corps 12 et qui délimite un orifice de sortie 49 de la chambre d'expansion 22.

Selon un exemple de réalisation préféré, le corps 12 est réalisé par moulage en injection de matière plastique.

Aussi, selon la figure 2, le dispositif 10 est équipé d'une membrane 50 déformable élastiquement de forme globalement en T, qui comprend un disque 52 annulaire radial qui est interposé axialement entre la collerette supérieure 40 du réservoir 24 et le siège 48 formé par le corps 12, et une partie tubulaire 54 qui s'étend axialement dans le réservoir 24, depuis le disque 52 annulaire.

La partie tubulaire 54 de la membrane 50 est conçue pour s'étendre axialement, sous l'effet de la pression du gaz généré par le générateur de gaz 16, pour pousser le piston supérieur 32 vers le bas afin d'éjecter le principe actif 26 à travers la buse 28 d'injection.

A cet effet, la membrane 50 est réalisée dans un matériau à base d'élastomère. Plus particulièrement, la membrane 50 est réalisée en EPDM, c'est-à-dire en éthylène-propylène-diène monomère.

En référence à la figure 1, le corps 12 est enveloppé par un capot 56 creux qui délimite une ouverture inférieure fermée par une semelle 58 horizontale formant fond de capot.

La semelle 58 délimite un passage circulaire 60 autour de l'axe B d'injection qui est adapté pour le passage de la buse 28 d'injection et de l'extrémité inférieure du corps 12, de sorte que la buse 28 comporte un tronçon inférieur faisant saillie verticalement vers le bas hors du capot 56.

Aussi, le dispositif 10 d'injection est équipé d'un bouchon 62 qui est monté sur le corps 12 de façon amovible par un moyen de verrouillage du type à baïonnette.

La buse 28 est vissée sur une extrémité libre débouchante du logement 44 formé par le corps 12, la buse 28 comprimant axialement l'ensemble formé par le réservoir 24 et la membrane 50 sur le siège 48 du logement 44.

Aussi, la buse 28 d'injection, illustrée en détail à la figure 3, présente une forme globalement cylindrique qui s'étend axialement suivant l'axe B d'injection depuis une face supérieure 64 en appui axial sur la face inférieure 38 du réservoir 24, jusqu'à une face inférieure 66 d'injection adaptée pour coopérer avec un opercule 68 de fermeture.

La face périphérique 70 cylindrique de la buse 28 présente un filetage 72 conçu pour visser la buse 28 sur l'extrémité libre du corps 12, le corps 12 étant équipé d'un taraudage 74 complémentaire prévu à cet effet.

De plus, la buse 28 délimite trois canaux axiaux d'injection (non représentés) qui s'étendent parallèlement à l'axe B d'injection, chaque canal débouchant dans la face supérieure 64 et dans la face inférieure 66 de la buse 28.

La buse 28 délimite un logement 76 central qui est adapté pour recevoir le piston inférieur 34 à la suite du déclenchement de l'injection.

Plus particulièrement, l'extrémité libre supérieure de chaque canal forme un évasement qui communique avec le logement 76, pour permettre au principe actif 26 de pénétrer dans chaque canal, depuis le logement, lorsque le piston inférieur 34 est tombé dans le logement 76.

En effet, lorsque le générateur de gaz 16 est déclenché, le gaz sous pression pousse la colonne de liquide formée par le piston supérieur 32, le principe actif 26 et le piston inférieur 34, le piston inférieur 34 tombant dans le logement 76 de la buse 28 prévu à cet effet pour permettre au principe actif 26 de s'écouler à travers les canaux.

Conformément à l'invention, en référence à la figure 4, le filetage 72 de la buse 28 présente une section globalement triangulaire, le filetage 72 étant délimité par une face supérieure 78 qui présente un premier angle A1 par rapport à un axe C radial transversal perpendiculaire à l'axe d'injection B, et une face inférieure 80 qui présente un second angle A2 par rapport à l'axe C radial.

Comme on peut le voir à la figure 4, le premier angle A1 est supérieur au second angle A2.

Le filetage 72 de la buse 28 est du type en dent de scie, c'est-à-dire que le second angle A2 formé par la face inférieure 80 de la buse est non nul de sorte que la face inférieure 80 forme une face de dépouille.

En effet, la buse 28 d'injection est réalisée de préférence en moulage par injection de matière plastique et la face inférieure 80 forme une face de dépouille permettant le démoulage de la buse 28 de son moule de fabrication.

Plus particulièrement, le premier angle A1 est d'environ 35 degrés et le second angle A2 est d'environ 10 degrés.

Ainsi, l'angle délimité entre la face supérieure 78 et la face inférieure 80 du filetage 72 est d'environ 45 degrés.

Selon la figure 3, la face inférieure 80 du filetage 72 de la buse 28 est en appui sur une face supérieure 82 complémentaire du taraudage 74 associé formé par le corps 12, pour reprendre les efforts axiaux de poussée transmis par la buse 28 au cours de l'injection.

Selon un exemple de réalisation préféré, la portion courbe R qui relie deux triangles voisins formés par le filetage 72 présente un rayon de courbure d'environ 0,11 millimètre. On entend par portion courbe R la portion en creux qui relie la face inférieure 80 d'un premier triangle et la face supérieure 78 d'un second triangle voisin du filetage 72 de la buse 28.

Aussi, le pas du filetage 72 est d'environ un millimètre.

Le dispositif 10 d'injection selon l'invention présente plusieurs avantages.

De part la forme du filetage 72 en dent de scie, les efforts transmis sur le corps 12 sont proche de la verticale, plus particulièrement ces efforts sont perpendiculaires à la face inférieure 80 du filetage 72 formant face d'appui, c'est-à-dire que les efforts sont proches d'un angle de 10 degrés par rapport à un axe vertical.

A l'inverse, selon l'art antérieur, le filetage 72 est généralement un filetage métrique, ou filetage isométrique, c'est-à-dire un filetage en section en triangle équilatéral qui est délimité par une face supérieure qui présente un premier angle proche de trente degrés et, et une face inférieure qui présente un second angle proche de trente degrés également.

Ainsi, le dispositif 10 selon l'invention permet de moins solliciter le corps 12 par des efforts radiaux, pour éviter de casser le corps 12.

De plus, le dispositif selon l'invention permet un couple de serrage important de la buse 28 sur le corps 12, ce qui permet de comprimer axialement suffisament le disque 52 annulaire de la membrane 50 contre le siège 48 formé par le corps 12, afin d'éviter les fuites à ce niveau.

## Revendications

1. Dispositif (10) d'injection sans aiguille comportant :
- un corps (12) formant un logement (44) qui s'étend axialement depuis un siège (48) radial supérieur, suivant un axe (B) d'injection,
- un générateur de gaz (16),
- un réservoir (24) tubulaire qui contient un principe actif (26) et qui s'étend axialement dans ledit logement (44) depuis une extrémité supérieure (40), jusqu'à une extrémité inférieure (36), et
- une buse (28) d'injection du principe actif (26) qui est agencée à l'extrémité inférieure du réservoir (24), et qui présente un filetage (72) externe adapté pour être vissé sur un taraudage (74) complémentaire formé par le corps (12),
le dispositif (10) d'injection étant **caractérisé**
**en ce qu'**il comprend une membrane (50) comprenant un disque (52) annulaire radial qui est interposé axialement entre ladite extrémité supérieure (40) et ledit siège radial supérieur (48), et une partie tubulaire (54) qui s'étend axialement dans le réservoir (24), depuis le disque annulaire (52), et
**en ce que** le filetage (72) de la buse (28) présente une section globalement triangulaire, ledit filetage (72) étant délimité par une face supérieure (78) qui présente un premier angle (A1) par rapport à un axe (C) radial perpendiculaire à l'axe (B) d'injection, et une face inférieure (80) qui présente un second angle (A2) par rapport audit axe (C) radial, et en ce que le premier angle (A1) est supérieur au second angle (A2), le filetage (72) étant du type en dent de scie, le second angle (A2) formé par la face inférieure (80) de la buse (28) étant non nul de sorte que ladite face inférieure (80) forme une face de dépouille.

2. Dispositif (10) d'injection sans aiguille selon la revendication 1, **caractérisé en ce que** le premier angle (A1) est d'environ 35 degrés.

3. Dispositif (10) d'injection sans aiguille selon l'une quelconque des revendications précédentes, **caractérisé en ce que** le second angle (A2) est d'environ 10 degrés.

4. Dispositif (10) d'injection sans aiguille selon l'une quelconque des revendications précédentes, **caractérisé en ce que** la face inférieure (80) du filetage (72) de la buse (28) est en appui sur une face supérieure (82) complémentaire du taraudage (74) associé formé par le corps (12), pour reprendre les efforts axiaux de poussée transmis par la buse (28) au cours de l'injection.

5. Dispositif (10) d'injection sans aiguille selon l'une quelconque des revendications précédentes, **caractérisé en ce que** la portion courbe (R) qui relie deux triangles voisins formés par le filetage 72 présente un rayon de courbure d'environ 0,11 millimètre.

6. Dispositif (10) d'injection sans aiguille selon l'une quelconque des revendications précédentes, **caractérisé en ce que** le pas du filetage (72) est d'environ un millimètre.

7. Dispositif (10) d'injection sans aiguille selon l'une quelconque des revendications précédentes, **caractérisé en ce que** la buse (28) d'injection est réalisée en moulage par injection.

8. Dispositif (10) d'injection sans aiguille selon l'une quelconque des revendications précédentes, **caractérisé en ce que** le corps (12) est réalisé en moulage par injection.

9. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadellose Injektionsvorrichtung (10), umfassend :
- einen Körper (12), der eine Aufnahme (44) bildet, die sich axial von einem oberen radialen Sitz (48) entlang einer Injektionsachse (B) erstreckt,
- einen Gasgenerator (16),
- einen röhrenförmigen Behälter (24), der einen Wirkstoff (26) enthält und sich axial in der Aufnahme (44) von einem oberen Ende (40) bis zu einem unteren Ende (36) erstreckt, und
- eine Injektionsdüse (28) des Wirkstoffs (26), die am unteren Ende des Behälters (24) angeordnet ist und ein Außengewinde (72) aufweist, das geeignet ist, um in ein vom Körper (12) gebildetes ergänzendes Innengewinde (74) geschraubt zu werden,
wobei die Injektionsvorrichtung (10) **dadurch gekennzeichnet ist,**
**dass** sie eine Membran (50) umfasst, die eine radiale ringförmige Scheibe (52) umfasst, die axial zwischen dem oberen Ende (40) und dem oberen radialen Sitz (48) eingelegt ist, und einen röhrenförmigen Teil (54), der sich axial von der ringförmigen Scheibe (52) aus im Behälter (24) erstreckt, und
wobei das Gewinde (72) der Düse (28) einen insgesamt dreieckigen Querschnitt aufweist, wobei das Gewinde (72) durch eine Oberseite (78) begrenzt ist, die einen ersten Winkel (A1) in Bezug auf eine radiale Achse (C) senkrecht zur Injektionsachse (B) aufweist, und eine Unterseite (80), die einen zweiten Winkel (A2) in Bezug auf die radiale Achse (C) aufweist, und dadurch, dass der erste Winkel (A1) größer als der zweite Winkel (A2) ist, wobei das Gewinde (72) vom Typ Sägezahn ist, wobei der zweite Winkel (A2), der von der Unterseite (80) der Düse (28) gebildet wird, ungleich Null ist, sodass die Unterseite (80) eine Freifläche bildet.

2. Nadellose Injektionsvorrichtung (10) nach Anspruch 1, **dadurch gekennzeichnet, dass** der erste Winkel (A1) etwa 35 Grad beträgt.

3. Nadellose Injektionsvorrichtung (10) nach einem der vorstehenden Ansprüche, **dadurch gekennzeichnet, dass** der zweite Winkel (A2) etwa 10 Grad beträgt.

4. Nadellose Injektionsvorrichtung (10) nach einem der vorstehenden Ansprüche, **dadurch gekennzeichnet, dass** die Unterseite (80) des Gewindes (72) der Düse (28) an einer ergänzenden Oberseite (82) des durch den Körper (12) gebildeten zugehörigen Innengewindes (74) anliegt, um die von der Düse (28) beim Injizieren übertragenen axialen Schubkräfte aufzunehmen.

5. Nadellose Injektionsvorrichtung (10) nach einem der vorstehenden Ansprüche, **dadurch gekennzeichnet, dass** der gekrümmte Abschnitt (R), der zwei benachbarte Dreiecke, die durch das Gewinde 72 gebildet werden, verbindet, einen Krümmungsradius von etwa 0,11 Millimetern aufweist.

6. Nadellose Injektionsvorrichtung (10) nach einem der vorstehenden Ansprüche, **dadurch gekennzeichnet, dass** die Steigung des Gewindes (72) etwa einen Millimeter beträgt.

7. Nadellose Injektionsvorrichtung (10) nach einem der vorstehenden Ansprüche, **dadurch gekennzeichnet, dass** die Injektionsdüse (28) im Spritzgussverfahren gefertigt ist.

8. Nadellose Injektionsvorrichtung (10) nach einem der vorstehenden Ansprüche, **dadurch gekennzeichnet, dass** der Körper (12) im Spritzgussverfahren gefertigt ist.

9. Nadellose Injektionsvorrichtung (10) nach einem der vorstehenden Ansprüche, **dadurch gekennzeichnet, dass** der im Behälter (24) enthaltene Wirkstoff (26) aus der Gruppe ausgewählt ist, welche die folgenden Wirkstoffe umfasst:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazinhydrochlorid,
- Zuclopenthixolacetat,
- Danaparoid-Natrium,
- Enoxaparin-Natrium,
- Estradiolcypionat,
- Medoxyprogesteronacetat,
- Medroparin-Calcium,
- Methylprednisolonacetat,
- Heparin-Calcium,
- Terbulin.

## Claims

1. A needleless injection device (10) including:
- a body (12) forming a housing (44) which extends axially from an upper radial seat (48), along an injection axis (B),
- a gas generator (16),
- a tubular reservoir (24) which contains an active ingredient (26) and which extends axially in said housing (44) from an upper end (40) to a lower end (36), and
- a nozzle (28) for injecting the active ingredient (26) which is arranged at the lower end of the reservoir (24), and which has an external thread (72) adapted to be screwed onto a complementary internal thread (74) formed by the body (12),
the injection device (10) being **characterized**
**in that** it comprises a membrane (50) comprising a radial annular disc (52) which is interposed axially between said upper end (40) and said upper radial seat (48), and a tubular part (54) which extends axially into the reservoir (24), from the annular disc (52), and
**in that** the thread (72) of the nozzle (28) has a generally triangular section, said thread (72) being delimited by an upper face (78) which has a first angle (A1) with respect to a radial axis (C) perpendicular to the injection axis (B), and a lower face (80) which has a second angle (A2) with respect to said radial axis (C), and in that the first angle (A1) is greater than the second angle (A2), the thread (72) being of the sawtooth type, the second angle (A2) formed by the lower face (80) of the nozzle (28) being non-zero so that said lower face (80) forms a flank.

2. The needleless injection device (10) according to claim 1, **characterized in that** the first angle (A1) is approximately 35 degrees.

3. The needleless injection device (10) according to any one of the preceding claims, **characterized in that** the second angle (A2) is approximately 10 degrees.

4. The needleless injection device (10) according to any one of the preceding claims, **characterized in that** the lower face (80) of the thread (72) of the nozzle (28) bears on a complementary upper face (82) of the associated internal thread (74) formed by the body (12), to take up the axial thrust forces transmitted by the nozzle (28) during injection.

5. The needleless injection device (10) according to any one of the preceding claims, **characterized in that** the curved portion (R) which connects two neighboring triangles formed by the thread 72 has a radius of curvature of approximately 0.11 millimeters.

6. The needleless injection device (10) according to any one of the preceding claims, **characterized in that** the pitch of the thread (72) is approximately one millimeter.

7. The needleless injection device (10) according to any one of the preceding claims, **characterized in that** the injection nozzle (28) is made by injection molding.

8. The needleless injection device (10) according to any one of the preceding claims, **characterized in that** the body (12) is made by injection molding.

9. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.
